# EUROPEAN PATENT APPLICATION

(11) **EP 1 023 916 A1**
(43) Date of publication of application: **02.08.2000**
(21) Application number: 99830033.9
(22) Date of filing: 28.01.1999
(51) Int. Cl.: A61N 1/362

(54) **Implantable cardiostimulation apparatus**

(71) Applicant: SORIN BIOMEDICA CARDIO S.p.A., 13040 Saluggia (Vercelli) (IT)
(72) Inventor: Plicchi, Gianni, 40136 Bologna (IT); Garberoglio, Bruno, 10156 Torino (IT); Gaggini, Guido, 20128 Milano (IT); Marcelli, Emanuela, 62100 Macerata (IT)
(74) Representative: Bosotti, Luciano

(57) **Abstract**

The implantable heart-stimulation apparatus comprises:
- a plurality of stimulation electrodes (1 to n) which can be applied to the heart muscle in one or more chambers of the heart in order to apply electrical stimulation signals thereto, and
- stimulation-control means (11 to 14, 21 to 24, 3, 4) for applying stimulation signals to the electrodes (1 to n) in accordance with a time plan determined individually and separately for each electrode of the plurality (1 to n).

The preferred application is for the correction of myocardial electrical conduction defects, for example, in atrial fibrillation and cardiac insufficiency.

## Description

The present invention relates, in general, to heart-stimulation techniques. In particular, the invention has been developed with a view to its possible application in improving the electro-mechanical characteristic of the function of the atria and the ventricles and/or in preventing arrhythmic events such as fibrillation also resulting from pathological anomalies of the conduction rate and of the duration of the refractory periods.

Human atrial fibrillation probably involves at least a limited establishment of interlaced re-entrant wavelets or the establishment of ectopic rapid-discharge foci, both of which phenomena can be treated by radio-frequency ablation. The wavelength of the re-entry circuit is related to the rate of conduction and to the refractory period. Both of these parameters in turn depend on any drugs administered, on the heart rate and on atrial myotasis, on autonomic influences, and/or on the anisotropy of the myocardium. The functional circuit which can induce atrial stimulation within the atrium is therefore not stable in space or in time; it can be altered by electrical stimulation so as to prevent or to render more difficult the possible production of arrhythmia. In this connection, the use of atrial stimulation techniques in several sites has already been proposed. In particular, at least two different techniques have been proposed, as proved, for example, by the following works:

"Biatrial pacing and AF prevention" by J. C. Daubert, G. R. d'Allones, and P. Mabo in Working Group on Arrhythmias of the European Society of Cardiology; International meeting; Atrial fibrillation, Bologna, Italy - 12, 13, 14 October 1997, and "Prevention of recurrent atrial fibrillation with chronic dual site right atrial pacing" by S. Saksena, A. Prakash, M. Hill et al. in J. Am. Coll. Cardiol. 1996, 28:687-694.

In the technique described by Daubert et al, the leads are situated in the upper right atrium as well as in the central or distal portion of the coronary sinus in order to perform a selective stimulation and sensing action in the left atrium whereas, in the technique described by Saksena et al, the top electrode of the second lead is fixed to the edge of the ostium of the coronary sinus in a rear and lower position in the right atrium.

In the technique of Daubert et al, the two atrial leads are connected to the atrial connector or port of a bipolar DDD pacemaker by means of a Y-shaped adaptor, whereas in the technique of Saksena et al, the device is a normal DDD pacemaker and simultaneous stimulation in the two atrial sites is possible only in stimulated atrial cycles.

Another pathological situation which frequently arises in cardiac insufficiency, particularly where there is dilation and obstruction, consists of asynchronism of the contraction of the ventricular chambers caused precisely by an altered rate of conduction towards the two ventricles of the stimuli generated physiologically by stimulation. The asynchronism of these contractions produces an actual antagonistic effect between the deformations of the muscle masses of the two ventricles, which is revealed by a considerable reduction in heart performance or by actual muscular obstructions to the left-ventricle ejection stage.

At present the sole, incomplete approach to this problem consists of so-called simultaneous biventricular stimulation in which the stimulus is sent simultaneously to both of the two heart chambers in question by the positioning of two electrodes therein.

The object of the present invention is to overcome the intrinsic limitations of the solutions described above.

According to the present invention, this object is achieved by means of a heart-stimulation device having the specific characteristics recited in the following claims.

In the currently-preferred embodiment, the heart-stimulation apparatus according to the invention is characterized by electrodes which are inserted permanently in the heart chambers and which can electrically stimulate the myocardium and detect the electro-physiological signal individually and locally.

The determination of the number of electrodes or poles, as well as the number of leads on which they are arranged, and also the selection of the anatomical sites in which to locate them, depend on the patient's specific arrhythmic condition. This is because the primary object of the device is to regularize the electro-physiological conduction processes in the individual heart chamber and/or between the various heart chambers, which object cannot be achieved by the known solutions which comprise a simultaneous stimulation of different regions.

For this purpose, the stimulator can be programmed so as to select, for example, suitable time sequences of stimuli which can be delivered by the various poles, and to receive electro-physiological signals in accordance with a time plan programmable individually for each pole, with the particular object of determining time sequences of progressive activation within a heart chamber or between several heart chambers, so as to reproduce or alter the natural rate of propagation of the spontaneous excitation.

The object of this type of stimulation is to improve the electro-mechanical characteristic of the function of the heart chambers (preventing, for example, competition between different chambers) and/or to prevent arrhythmic events, such as fibrillation also resulting from pathological anomalies of the conduction rate and of the duration of the refractory periods.

In a particularly preferred embodiment, the stimulator according to the invention is of the dual-chamber type in which the atrial stimulation and sensing lead is a multi-pole lead which can deliver pulses in accordance with a time plan programmable individually for each pole in order to bring about a progressive activity of the chamber such as to simulate normal electro-physiological conduction and to correct the anomalies of the rate of conduction of the local refractory periods of the myocardium which give rise to supraventricular arrhythmias.

Another particularly preferred embodiment consists of the execution of a selective stimulation of the right ventricle and of the left ventricle with a capability for suitable programming of the sequence and of the time interval both of the stimulation of the two ventricles and of the atrio-ventricular delays.

The duration of the sequence of stimulation pulses, for example, atrial stimulation pulses (to prevent fibrillation) is programmable and can be deduced by time analysis of the electro-physiological signals recorded by the stimulator at the poles during the stages in which arrhythmia is absent, or by a single-pole electro-stimulation stage, or can even be estimated from the spatial positioning of the poles.

In the case of multi-site biventricular stimulation, the intervals of the stimulation sequence can be optimized by examination of the mechanical heart function deduced by external methods (e.g. by echocardiogramme) or by special sensors included in the implantable device, for example, of the type described in US-A-5 304 208, US-A-5 496 351, or US-A-5 609 612.

The atrial stimulation sequence itself is activated in synchronism with the atrial electrogram detected by the pole nearest to the node of the atrial sinus or in an automatic and asynchronous manner regulated by the programming of the atrial stand-by of the stimulator also comprising the rate-responsive function.

The sequence of activation of the poles is programmable and does not necessarily follow their geometric progression for the purpose of a possible correction of the electro-physiological path of the atrial activation wavelets in the prevention of supraventricular arrhythmia.

The activation of each individual pole involves the determination of a programmable blanking interval in all of the remaining poles.

In the case of an atrial stimulation sequence synchronous with the spontaneous activity, the regulation of the duration of the atrio-ventricular delay (the AV delay) has the atrial electrogram as a reference, whereas, in the case of an asynchronous sequence, the reference is the last atrial stimulus.

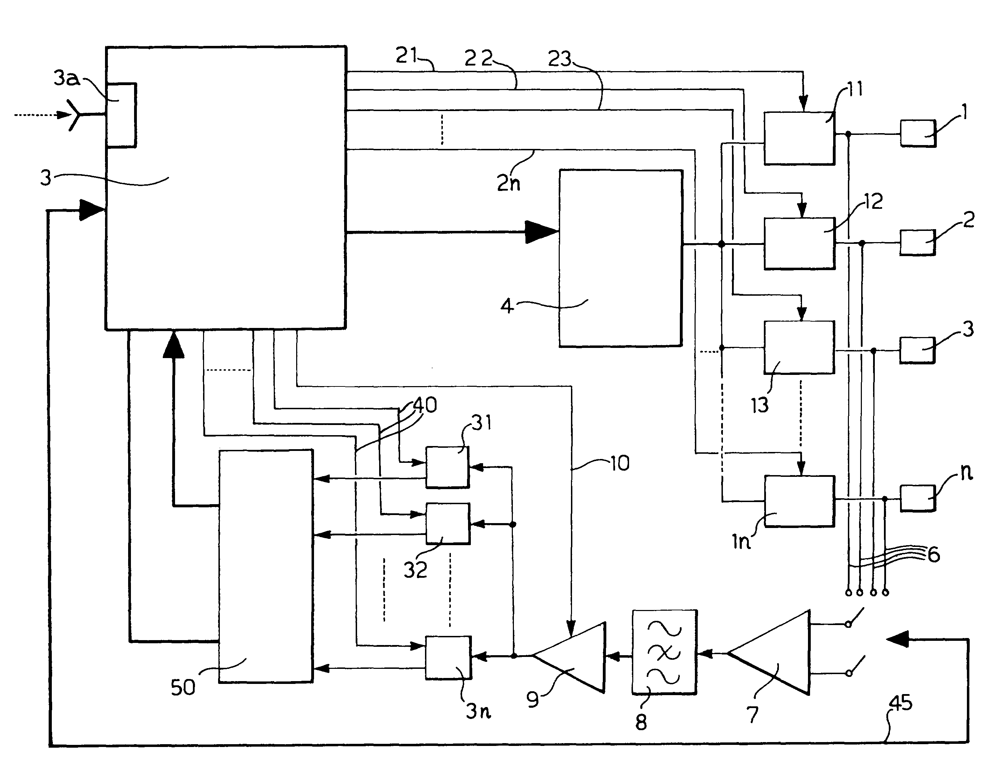

The invention will now be described, purely by way of nonlimiting example, with reference to the appended drawings, comprising a single drawing which shows the circuit structure of a multi-pole stimulator according to the invention, in the form of a block diagram.

In the appended drawing, a plurality of electrodes (or "poles") which can be fitted in sites in the heart suitable for bringing about a stimulation effect on the heart muscle by the application of electrical signals are indicated 1, 2, 3, ...n.

The structural characteristics of these electrodes, with regard, for example, to the selection of the constituent materials, any surface treatment, etc., correspond substantially to those currently used in known implantable defibrillators.

In particular, the term "electrode" or "pole" as used in the present description and, where applicable, in the following claims, is intended to define any electrically-conductive member which can be associated with the heart muscle in a relationship suitable for the transmission of an electrical signal. Consequently, the electrodes or poles in question may either be configured as physically separate members which are thus intended to be fitted in distinct and separate respective myocardial sites, or may be associated in groups or sets with respective supporting structures (so-called "leads") so as to be fitted in respective myocardial sites separately (in the sense that each electrode or pole can transfer - and detect - a respective signal to - and from - the myocardium) but not independently, since the electrodes or poles of each group disposed on a respective lead are implanted simultaneously as a result of the implantation of the respective supporting element.

With regard to the selection of the number n, the solution according to the invention can be implemented either in a minimal configuration comprising only two electrodes, or in more complex configurations with a number n equal, for example, to four, eight or ten (the selection of an even number is not, however, essential).

The electrodes 1 to n may either be identical to one another or may have different shapes according to the sites in which they are to be positioned.

The stage of the implantable stimulator in which the voltage pulses to be applied to the electrodes 1 to n via respective electronic enabling switches 11, 12, 13, ..., ln are generated is indicated 4.

The switches (or control gates) in question are enabled to operate (thus allowing the signals generated in the stage 4 to pass selectively towards the electrodes 1, 2, ...n) by respective enabling lines 21, 22, 23, ..., 2n controlled by a timing and control unit 3, preferably having an associated telemetering interface 3a of generally known type.

The control performed by the unit 3 by means of the lines 21, 22, 23, ...2n enables the characteristics of the stimulation signals applied to the myocardium to be programmed, particularly with regard to duration, amplitude, and polarity and to the ability to activate the pulses at the various electrodes 1, 2, 3, ...,n sequentially in time (in accordance with a typical time-sharing scheme).

As a result, the above-described multi-pole configuration permits, in particular, the creation of an electrical stimulation field with variable geometry designed for the patient's anatomy - with a high degree of flexibility - by virtue of the ability of the unit 3 to programme the emission of the control signals.

The stimulation may advantageously be activated by a sensing operation performed by the electrodes 1, 2, 3, ...n themselves.

Respective sensing lines, generally indicated 6, are provided for this purpose and enable the electrical signals indicative of the local myocardial activity at the respective implantation site to be detected by the respective electrode so as to supply to the unit 3 a set of signals which enables the unit to see and to identify the occurrence of time anomalies in the myocardial electrical conduction.

The diagram of the appended drawing shows a multiplexer, indicated 7, which enables the signal coming from a respective electrode 1, 2, 3, ...n, to be detected selectively by the respective line of the set indicated 6. After anti-noise filtering performed in a filter 8 and automatic gain control performed in an automatic gain-control circuit (AGC) 9 controlled by the unit 3 by means of a line 10, the sensing signals thus detected reach respective sampling circuits 31, 32, ..., 3n, preferably of the sample-and-hold type, which are controlled by the unit 3 by means of respective enabling lines (generally indicted 40) in a manner coordinated with the driving of the multiplexer 7 performed by the unit 3 by means of a line 45.

The samples stored in the circuits 31, 32, ...3n at any particular time (each relating to the sensing operation performed at the time in question by a respective electrode 1, 2, ... n) are sent to an analog/digital conversion circuit 50 for transmission to the unit 3.

The unit 3 can thus carry out a time analysis of the electro-physiological signals recorded in the electrodes 1, 2, ...n during stages in which arrhythmia is absent or after a single-pole electro-stimulation stage (possibly taking account of the spatial positioning of the poles) so as to be able to deliver the stimulation pulses in accordance with a time plan programmable individually for each electrode. This permits, for example, progressive activation of the chamber treated such as to simulate the normal electro-physiological conduction and to correct anomalies of the rate of conduction of the local refractory periods of the myocardium such as to give rise to supraventricular arrhythmias. Naturally, this relates to the prevention of atrial fibrillation situations.

In the case of cardiac insufficiency, on the other hand, the regulation of the activation times of the poles 1, 2, ...n is controlled by the unit 3 either in response to the implanted heart-function signal or on the basis of external haemodynamic parameters such as the cardiac output or the aorto-ventricular pressure gradient.

Clearly, the various electronic components shown in the drawing may be in the form of discrete blocks or components, or may be integrated in a single circuit.

Naturally, the principle of the invention remaining the same, the details of construction and forms of embodiment may be varied widely with respect to those described and illustrated, without thereby departing from the scope of the present invention as defined by the following claims.

## Claims

1. Implantable heart-stimulation apparatus, characterized in that it comprises:
- a plurality of stimulation electrodes (1 to n) which can be applied to the heart muscle in order to apply electrical stimulation signals thereto, and
- stimulation-control means (11 to 14, 21 to 24, 3, 4) for applying stimulation signals to the electrodes (1 to n) in accordance with a time plan determined individually and separately for each electrode of the plurality (1 to n).

2. Apparatus according to Claim 1, characterized in that the stimulation-control means (11 to 14, 21 to 24, 3, 4) generate the stimulation signals in accordance with a time plan determined in dependence on at least one parameter selected from the group constituted by:
- time analysis of the electro-physiological signals recorded on the electrodes (1 to n),
- an electro-stimulation stage in at least one of the electrodes (1 to n),
- an estimate of the spatial positioning of the electrodes (1 to n),
- heart functionality parameters detected externally or by implanted sensors,
- aorto-ventricular pressure gradients.

3. Apparatus according to Claim 1 or Claim 2, characterized in that the stimulation-control means (11 to 14, 21 to 24, 3, 4) activate a given stimulation sequence synchronously with an electrogram detected by one of the electrodes (1 to n).

4. Apparatus according to Claim 3, characterized in that the said one of the electrodes is the electrode of the plurality (1 to n) which can be located in a position closest to the node of the atrial sinus, and in that the stimulation sequence is an atrial stimulation sequence.

5. Apparatus according to Claim 1, characterized in that the plurality of electrodes (1 to n) comprises electrodes which can be associated with different heart chambers, and in that the stimulation-control means (11 to 14, 21 to 24, 3, 4) can apply stimulation signals separated from one another in time to the electrodes which can be associated with different chambers, the separation in time being determined in dependence on the optimization of haemodynamic parameters derived externally or by means of implantable sensors.

6. Apparatus according to Claim 1, characterized in that the stimulation-control means (11 to 14, 21 to 24, 3, 4) activate a given stimulation sequence automatically and asynchronously in dependence on the stimulator stand-by programming.

7. Apparatus according to any one of the preceding claims, characterized in that the stimulation-control means (11 to 14, 21 to 24, 3, 4) comprise a rate-responsive function.

8. Apparatus according to any one of the preceding claims, characterized in that the stimulation-control means (11 to 14, 21 to 24, 3, 4) apply the stimulation signals to the electrodes (1 to n) in accordance with a sequence of the electrodes (1 to n) subjected to stimulation which sequence can be determined selectively independently of the geometrical location of the electrodes (1n).

9. Apparatus according to any one of the preceding claims, characterized in that it further comprises detector means (7 to 10, 31 to 3n, 40, 50) for transferring to the stimulation-control means (3, 4) respective sensing signals derived from the electrodes (1 to n), and in that the application of a stimulation signal to one of the electrodes (1n) brings about a refractory state (blanking) of the detector means.

10. Apparatus according to any one of Claims 1 to 9, characterized in that it comprises detector means (7 to 10, 31 to 3n, 40, 50) for transferring to the stimulation-control means (3, 4) respective electro-physiological sensing signals derived separately from the electrodes (1 to n) of the plurality, in accordance with a programmable time plan.

11. Apparatus according to any one of the preceding claims, characterized in that at least some of the electrodes (1 to n) of the plurality can be implanted separately and independently of the other electrodes of the plurality.

12. Apparatus according to any one of the preceding claims, characterized in that at least some of the electrodes (1 to n) of the plurality are arranged in at least one respective set of electrodes carried by a common support for combined implantation.
